# EUROPÄISCHE PATENTSCHRIFT

(11) **EP 3 611 527 B1**
(45) Veröffentlichungstag und Bekanntmachung des Hinweises auf die Patenterteilung: **14.07.2021**
(21) Anmeldenummer: 18189310.8
(22) Anmeldetag: 16.08.2018
(51) Int. Cl.: G01R 33/36

(54) **LOKALSPULE UND SYSTEM ZUR DRAHTLOSEN ENERGIEÜBERTRAGUNG**
LOCAL COIL AND SYSTEM FOR WIRELESS ENERGY TRANSMISSION
BOBINE LOCALE ET SYSTÈME DE TRANSMISSION D'ÉNERGIE SANS FIL

(43) Veröffentlichungstag der Anmeldung: 19.02.2020
(73) Patentinhaber: Siemens Healthcare GmbH, 91052 Erlangen (DE)
(72) Erfinder: Fackelmeier, Andreas, 91177 Thalmässing (DE)

(56) Entgegenhaltungen:
- WO-A1-2018/060332
- JP-A- 2017 046 797
- US-A1- 2009 237 079
- US-A1- 2014 218 035

## Beschreibung

Die Erfindung betrifft eine Lokalspule und ein System aus Lokalspule und Energiesendeeinrichtung zur drahtlosen Energieversorgung der Lokalspule.

Magnetresonanztomographen sind bildgebende Vorrichtungen, die zur Abbildung eines Untersuchungsobjektes Kernspins des Untersuchungsobjektes mit einem starken äußeren Magnetfeld ausrichten und durch ein magnetisches Wechselfeld zur Präzession um diese Ausrichtung anregen. Die Präzession bzw. Rückkehr der Spins aus diesem angeregten in einen Zustand mit geringerer Energie wiederum erzeugt als Antwort ein magnetisches Wechselfeld, auch als Magnetresonanzsignal bezeichnet, das über Antennen empfangen wird.

Mit Hilfe von magnetischen Gradientenfeldern wird den Signalen eine Ortskodierung aufgeprägt, die nachfolgend eine Zuordnung von dem empfangenen Signal zu einem Volumenelement ermöglicht. Das empfangene Signal wird dann ausgewertet und eine dreidimensionale bildgebende Darstellung des Untersuchungsobjektes bereitgestellt. Die erzeugte Darstellung gibt eine räumliche Dichteverteilung der Spins an.

Um das Signal-zu-Rauschverhältnis zu verbessern, und auch um durch Parallel-Abtastung die Bilderfassung zu beschleunigen, werden zunehmend möglichst viele Empfangsantennen in Form einer als Lokalspulenmatrix bezeichneten Antennenmatrix möglichst nahe am Körper des Patienten angeordnet. Zur Übertragung der in der Lokalspule bzw. Lokalspulenmatrix empfangenen Signale wird üblicherweise eine Kabelverbindung verwendet. Dabei wirken die Kabel aber auch als Antennen während des Anregungspulses, sodass spezielle Sicherheitsmaßnahmen wie Mantelwellensperren vorgesehen werden müssen, um den Patienten nicht zu gefährden. Auch ist die Handhabung der Kabel umständlich.

Es sind auch schon Lokalspulen verfügbar, die die empfangenen Magnetresonanzsignale umsetzen oder digitalisieren und an den Magnetresonanztomographen senden. Zur Versorgung der Vorverstärker und Umsetzer sind jedoch Batterien erforderlich, die nur eine begrenzte Zeit überbrücken können und so einen kontinuierlichen Betrieb des Magnetresonanztomographen behindern.

Bei Verwendung drahtloser Energieübertragung können zum einen Magnetresonanzsignale gestört werden, zum anderen werden oft mehrere Lokalspulen am Patient angebracht, um keinen Wechsel während der Untersuchung vornehmen zu müssen und wertvolle Untersuchungszeit am Magnetresonanztomographen zu verschwenden. Die drahtlose Energieübertragung der Lokalspulen kann sich dabei gegenseitig beeinflussen und stören.

Aus der Druckschrift DE 10 2012 210 507 B4 ist eine Lokalspule für ein Magnetresonanzbildgebungssystem und ein Magnetresonanzbildgebungssystem bekannt, wobei die Lokalspule bzw. das Magnetresonanzbildgebungssystem zur drahtlosen Übertragung von Betriebsenergie bzw. eines Referenzsignals ausgebildet sind.

Die Druckschrift JP 2017 046797 A offenbart eine Lokalspule mit drahtloser Energieversorgung. Die Lokalspule weist einen ersten drahtlosen Verbindungsbaustein zur Übertragung eines Batterieladezustandes und einen zweiten drahtlosen Verbindungsbaustein zur drahtlosen Übertragung von Magnetresonanzsignalen auf.

Aus der Druckschrift US 2014/0218035 A1 ist ein Magnetresonanztomograph mit einer Energieübertragungseinrichtung bekannt. Die Energieübertragungseinrichtung überträgt Energie drahtlos zu einer Lokalspule durch resonante magnetische Kopplung.

Die Druckschrift WO 2018/060332 A1 beschreibt ein Magnetresonanzsystem mit einem Sensor zur Detektion einer drahtlosen Energieübertragung und eine drahtlose Energieaufnahmeeinrichtung. Der Sensor erkennt eine resonante magnetische Energieübertragung und die Vorrichtung zur Energieaufnahmevorrichtung nimmt in Abhängigkeit von dem Erkennen durch den Sensor Energie magnetisch auf.

Es ist eine Aufgabe der vorliegenden Erfindung, eine einfach handzuhabende Lokalspule bereitzustellen.

Die Aufgabe wird durch ein erfindungsgemäßes System nach Anspruch 1 gelöst.

Die nicht beanspruchte Lokalspule weist eine Energieversorgungseinrichtung auf. Die Energieversorgungseinrichtung ist ausgelegt, Verbraucher bzw. aktive Komponenten der Lokalspule im Betrieb mit Energie zu versorgen. Aktive Komponenten sind beispielsweise Vorverstärker wie Low-Noise-Amplifier (LNA), Mischer, Oszillatoren, A/D-Wandler oder Sender zur Übertragung der empfangenen Magnetresonanzsignale zu dem Magnetresonanztomographen. Die Energieversorgungseinrichtung ist vorzugsweise ausgelegt, die vollständige Übertragung aller Magnetresonanzsignale zumindest einer Bilderfassung sicherzustellen. Die Energieversorgungsvorrichtung weist deshalb auch einen Energiespeicher auf, der ausgelegt ist, die Übertragung auch bei Unterbrechungen oder zeitlichen Pausen der drahtlosen Energieversorgung sicherzustellen. Beispielhafte Energiespeicher sind nachfolgend in den Unteransprüchen angegeben.

Weiterhin weist die Energieversorgungseinrichtung eine Energieempfangseinrichtung auf, die ausgelegt ist, drahtlos Energie von einer externen Energiesendeeinrichtung aufzunehmen, um die Verbraucher der Lokalspule in Betrieb zu halten. Unterbrechungen in der drahtlosen Energieübertragung werden dabei durch den Energiespeicher überbrückt, wobei die Energieempfangseinrichtung zwischen diesen Unterbrechungen den Energiespeicher wieder lädt. Eine Energieempfangseinrichtung kann beispielsweise eine Spule sein, die durch Induktion Energie von der Energiesendeeinrichtung im Nahfeld empfängt, ohne dass eine elektromagnetische Welle in größerem Umfang in den freien Raum abgestrahlt wird. Ohne Abstrahlung in größerem Umfang ist dabei so zu verstehen, dass ein Anteil kleiner als 50%, 25 %, 10%, 5% oder 1 % der übertragenen Energie in den Raum abgestrahlt wird.

Weiterhin weist die Lokalspule eine Taktsteuerung auf. Die Taktsteuerung ist ausgelegt, sich mit einer externen Taktquelle zu synchronisieren, wie im Detail in den nachfolgenden Unteransprüchen ausgeführt ist. Die Taktsteuerung ist über eine Signalverbindung mit der Energieversorgungseinrichtung verbunden und ausgelegt, eine Energieaufnahme über die Energieempfangseinrichtung in Abhängigkeit von der Synchronisierung zu steuern. Unter Steuern ist insbesondere zu verstehen, dass die Taktsteuerung Zeitintervalle vorgibt, in denen die Energieversorgungseinrichtung drahtlos Energie aufnimmt. Unter Abhängigkeit ist insbesondere zu verstehen, dass der Zeitpunkt und/oder die Dauer des Zeitintervalls von der Synchronisierung abhängt.

Auf vorteilhafte Weise kann durch die Taktsteuerung und die Energieempfangseinrichtung vorbestimmt werden, wann die Lokalspule drahtlos Energie aufnimmt, sodass Wechselwirkungen zwischen mehreren Lokalspulen verhindert werden können.

In einer möglichen Ausführungsform der nicht beanspruchten Lokalspule weist die Taktsteuerung einen Synchronisierungseingang zum Synchronisieren mit einer externen Taktquelle auf. Denkbar ist es beispielsweise, dass die Taktsteuerung eine Antenne hat, um ein gesondertes Synchronisierungssignal zu empfangen. Möglich wäre es auch, dass die Taktsteuerung die Anregungspulse oder Gradientensignale der Magnetresonanztomographie als Synchronisationssignal nutzt. Auch eine interne Uhr der Taktversorgung, die nur in größeren Abständen mit dem Magnetresonanztomographen synchronisiert wird, beispielsweise in einer Ladeschale, wäre denkbar. Schließlich könnten Magnetresonanztomograph und Taktsteuerung auch durch eine externe dritte Taktquelle synchronisiert werden.

Auf vorteilhafte Weise erlaubt der Synchronisierungseingang eine Synchronisation von mehreren Lokalspulen und dem Magnetresonanztomographen, sodass diese gleichzeitig betrieben werden können, ohne sich bei der drahtlosen Energieversorgung gegenseitig zu stören oder die Energiesendeeinrichtung zu überlasten.

In einer denkbaren Ausführungsform der nicht beanspruchten Lokalspule ist der Synchronisierungseingang in Signalverbindung mit der Energieempfangseinrichtung. Der Synchronisierungseingang weist einen Demodulator auf, der ausgelegt ist, ein von der Energieempfangseinrichtung empfangenes externes Synchronsignal zu demodulieren und an die Taktsteuerung weiterzugeben.

Der Demodulator erlaubt auf vorteilhafte Weise ein Synchronisationssignal zu der Taktsteuerung der Lokalspule ohne zusätzliche Signalwege oder Radiofrequenzen zu übertragen, die den Betrieb stören könnten.

In einer denkbaren Ausführungsform der nicht beanspruchten Lokalspule ist die Taktsteuerung in Signalverbindung mit einer drahtlosen Kommunikationseinrichtung. Die Taktsteuerung oder die Energieversorgungseinrichtung ist dabei ausgelegt, eine Information über einen Ladezustand des Energiespeichers über die Kommunikationseinrichtung zu senden. Beispielsweise kann die Information gemeinsam mit einem MRT-Signal in Frequenzmultiplex, Zeitmultiplex und/oder digital übertragen werden. Es ist aber auch eine Übertragung mit einer unabhängigen bidirektionalen drahtlosen Übertragung für Steuerkommandos denkbar, die mit geringer Bandbreite arbeiten kann.

Auf vorteilhafte Weise ermöglicht eine Information über den Ladezustand, die Energieversorgung mehrerer Lokalspulen optimal zu koordinieren, sodass eine Energiesendeeinrichtung mit einer geringeren Leistung auskommen kann.

In einer möglichen Ausführungsform der nicht beanspruchten Lokalspule weist die Energieempfangseinrichtung der Lokalspule eine Induktionsspule mit einer Verstimmeinrichtung auf und die Taktsteuerung ist ausgelegt, die Verstimmeinrichtung zu steuern. Die Verstimmeinrichtung kann beispielsweise durch schaltbare Kapazitäten oder Induktivitäten realisiert sein. Denkbar sind auch Hochfrequenzschalter wie PIN-Dioden, die Kreis oder Leitung kurzschließen oder öffnen. Vorzugsweise ist die Induktionsspule ausgelegt, im magnetischen Wechselfeld einer entsprechenden Sendespule resonant durch Induktion Energie zu entnehmen. Vorzugsweise ist die Taktsteuerung ausgelegt, die Induktionsspule zu den Zeitintervallen zu verstimmen, in denen andere Taktsteuerungen anderer Lokalspulen deren Verstimmung zur drahtlosen Energieübertragung abschalten.

Auf vorteilhafte vermeidet die von der Taktsteuerung geschaltete Verstimmungseinrichtung eine Wechselwirkung mehrerer Induktionsspulen in unterschiedlichen Lokalspulen, was eine Verstimmung durch Kopplung mehrerer resonanter Schwingkreise und damit eine verschlechterte Energieübertragung verursachen würde.

In einer denkbaren Ausführungsform weist die nicht beanspruchte Lokalspule eine Kapazität, ein Supercap oder eine aufladbare Batterie als Energiespeicher auf. Es ist auch eine Kombination aus einem schnell ladbaren Energiespeicher und einem mit großer Kapazität denkbar, wie beispielsweise einem Supercap und einer Lithiumzelle, um eine kurze Ladezeit mit einer großen Kapazität zu verbinden.

Diese ladbaren Energiespeicher sind vorteilhafterweise in der Lage, die Energieversorgung der Lokalspule für eine kurze oder auch längere Unterbrechung der drahtlosen Energieübertragung sicherzustellen. Dabei sind Kapazitäten und Supercaps in sehr kurzer Zeit wieder aufgeladen, während Supercap und aufladbare Batterien auch längere Pausen der Energieübertragung überbrücken können.

Ein erfindungsgemäßes System weist eine Mehrzahl an Lokalspulen auf, eine Energiesendeeinrichtung und eine Taktquelle. Die Taktquelle ist ausgelegt, ein Taktsignal an die Taktsteuerungen der Lokalspulen zu senden. Das Taktsignal ist dabei so ausgelegt, dass nur eine echte Teilmenge der Lokalspulen gleichzeitig Energie über die Energieempfangseinrichtung aufnimmt. Mit anderen Worten stellt eine Kodierung in dem Taktsignal in Verbindung mit den Taktsteuerungen der Lokalspulen sicher, dass nur eine oder einige der Lokalspulen gleichzeitig drahtlos Energie empfangen. Beispielsweise kann das Taktsignal einen Zähler in einer Schleife aufweisen und die Lokalspulen individuelle Nummern, sodass nur bei Übereinstimmung von Zähler und Nummer eine Energieübertragung durch Deaktivieren der Verstimmungseinrichtung erfolgt.

In einer denkbaren Ausführungsform des erfindungsgemäßen Systems steht die Taktquelle in Signalverbindung mit der Energiesendeeinrichtung und ist ausgelegt, eine Energieaussendung der Energiesendeeinrichtung zu modulieren. Beispielsweise kann die Amplitude oder Frequenz moduliert werden, um einen Zähler digital kodiert zu übertragen.

Auf vorteilhafte Weise können so die Lokalspulen mit der Taktquelle ohne zusätzliche Signale synchronisiert werden.

Eine denkbare Ausführungsform eines Verfahrens zum Betrieb des Systems weist den Schritt auf, eine Synchroninformation durch die Taktquelle zu senden. Beispielsweise kann das Signal zur Energieübertragung moduliert werden oder eine drahtlose Schnittstelle zur Steuerung der Lokalspule genutzt werden.

In einem weiteren Schritt wird die Synchroninformation durch die Taktsteuerungen der Lokalspulen empfangen. Es kann dazu die Energieempfangseinrichtung beispielsweise ein zur Energieübertragung genutztes Signal demodulieren und so ein aufmoduliertes Synchronsignal an die Taktsteuerung über eine Signalverbindung weiterleiten. Möglich ist auch, dass zur Steuerung der Lokalspulen eine zusätzliche drahtlose Kommunikationsschnittstelle vorhanden ist, die das Synchronsignal empfängt und über eine Signalverbindung an die Taktsteuerung weiterleitet.

In einem anderen Schritt steuert die Energieversorgungseinrichtung über die Energieempfangseinrichtung die Energieaufnahme in Abhängigkeit von der Synchroninformation. Denkbar ist beispielsweise, dass die Energiesteuerung an einem durch die Synchroninformation bestimmten Zeitpunkt oder Dauer die Energieaufnahme ausführt.

Auf vorteilhafte Weise ermöglicht das erfindungsgemäße Verfahren mit dem erfindungsgemäßen System, die Energieaufnahme mehrerer Lokalspulen zu koordinieren und eine gleichmäßige Verteilung der Last zu erreichen.

In einer möglichen Ausführungsform des erfindungsgemäßen Verfahrens weist der Schritt der Energieaufnahme den Schritt auf, den Zustand der Verstimmeinrichtung zu ändern.

Auf vorteilhafte Weise wird so ermöglicht, dass die Induktionsspule nur während der Energieaufnahme auf das Signal abgestimmt ist, das die Energie drahtlos überträgt. Während der übrigen Zeit ist die induktionsspule hingegen verstimmt, sodass keine unerwünschten Wechselwirkungen wie ein Verstimmen durch Kopplung von unterschiedlichen Lokalspulen erfolgt und die Effizienz der Energieübertragung gemindert wird.

In einer denkbaren Ausführungsform des erfindungsgemäßen Verfahrens weist der Synchronisierungseingang von wenigstens einer der Lokalspulen einen Demodulator auf und steht in Signalverbindung mit der Energieempfangseinrichtung. Die Taktquelle des Systems steht in Signalverbindung mit der Energiesendeeinrichtung. Der Schritt des Sendens weist dabei den Schritt auf, die Energieaussendung der Energiesendeeinrichtung mit der Synchroninformation der Taktquelle zu modulieren und der Schritt des Empfangens den Schritt, das von der Energiesendeeinrichtung empfangene Signal mit dem Demodulator zu demodulieren und die empfangene Synchroninformation an die Taktsteuerung weiterzuleiten.

Die Übertragung der Synchroninformation mittels des zur Energieübertragung genutzten Signals erspart auf vorteilhafte Weise einen zusätzlichen drahtlosen Kommunikationsweg, der Störungen verursachen könnte.

In einer möglichen Ausführungsform des erfindungsgemäßen Verfahrens weist wenigstens eine der Lokalspulen eine drahtlose Kommunikationseinrichtung auf, die mit der Taktsteuerung in Signalverbindung steht. Das Verfahren weist den Schritt auf, eine Information über einen Ladezustand des Energiespeichers über die Kommunikationseinrichtung zu senden. Beispielsweise kann eine schmalbandige Kommunikationseinrichtung über Funk vorgesehen sein, mit der die drahtlose Lokalspule Einstellkommandos erhält und Statusinformationen sendet. Denkbar ist auch, dass die Information über die drahtlose Verbindung mit den Magnetresonanzsignalen zeitlich, in der Frequenz oder digital gemultiplext ist.

Auf vorteilhafte Weise kann so die Taktsteuerung das Laden der einzelnen Lokalspulen optimieren, sodass der Leistungsbedarf der Energiesendeeinrichtung und die SAR-Belastung minimiert werden.

In einer denkbaren Ausführungsform des erfindungsgemäßen Verfahrens empfängt die Taktquelle die Information zum Ladezustand in einem Schritt und ändert in dem Schritt des Sendens das ausgesendete Synchronsignal in Abhängigkeit von der Information über den Ladezustand.

Auf vorteilhafte Weise kann so die Taktquelle das Laden der Lokalspulen an den Ladezustand der Lokalspulen anpassen und diesen beschleunigen bzw. optimieren.

Die oben beschriebenen Eigenschaften, Merkmale und Vorteile dieser Erfindung sowie die Art und Weise, wie diese erreicht werden, werden klarer und deutlicher verständlich im Zusammenhang mit der folgenden Beschreibung der Ausführungsbeispiele, die im Zusammenhang mit den Zeichnungen näher erläutert werden.

Es zeigen:
- Fig. 1: eine beispielhafte schematische Darstellung eines Magnetresonanztomographen mit einer Antenne aus dem Stand der Technik;
- Fig. 2: eine beispielhafte schematische Darstellung einer Lokalspule aus dem Stand der Technik;
- Fig. 3: eine beispielhafte schematische Darstellung eines Magnetresonanztomographen mit einer Energiesendeeinrichtung;
- Fig. 4: einen schematischen Ablaufplan eines Verfahrens.

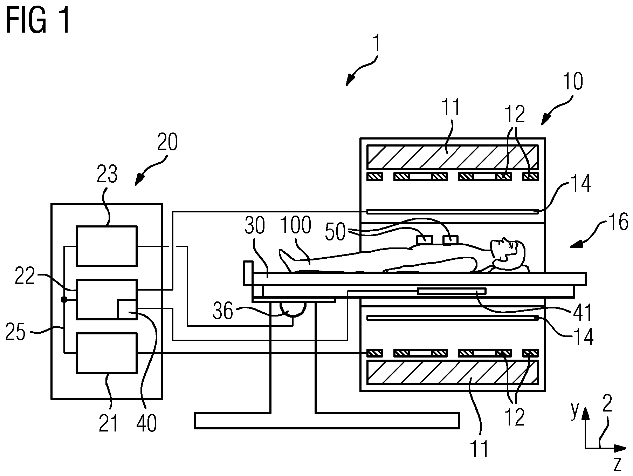

Fig. 1 zeigt eine schematische Darstellung eines Magnetresonanztomographen 1, der zusammen mit einem erfindungsgemäßen System zur Ausführung der Erfindung verwendet werden kann. Der in der Fig. 1 gezeigte Magnetresonanzteomograph 1 ist als solcher jedoch nicht Teil der Erfindung.

Die Magneteinheit 10 weist einen Feldmagneten 11 auf, der ein statisches Magnetfeld B0 zur Ausrichtung von Kernspins von Proben bzw. Patienten 100 in einem Aufnahmebereich erzeugt. Der Aufnahmebereich ist in einem Patiententunnel 16 angeordnet, der sich in einer Längsrichtung 2 durch die Magneteinheit 10 erstreckt. Ein Patient 100 ist mittels der Patientenliege 30 und der Verfahreinheit 36 der Patientenliege 30 in den Aufnahmebereich verfahrbar. Üblicherweise handelt es sich bei dem Feldmagneten 11 um einen supraleitenden Magneten, der magnetische Felder mit einer magnetischen Flussdichte von bis zu 3T, bei neuesten Geräten sogar darüber, bereitstellen kann. Für geringere Feldstärken können jedoch auch Permanentmagnete oder Elektromagnete mit normalleitenden Spulen Verwendung finden. Geringe Feldstärken sind insbesondere kostengünstiger zu realisieren und liefern mit modernen Verfahren zur Bilderfassung dennoch zufriedenstellende Ergebnisse. Dies sind beispielsweise Feldstärken von 1,5 Tesla, 1 Tesla oder auch 0,5 Tesla.

Die Magneteinheit 10 weist ebenfalls eine Körperspule 14 auf, die dazu ausgelegt ist, ein über eine Signalleitung zugeführtes Hochfrequenzsignal in das Untersuchungsvolumen abzustrahlen und von dem Patient 100 emittierte Resonanzsignale zu empfangen und über eine Signalleitung abzugeben. Bevorzugter Weise wird aber die Körperspule 14 für das Empfangen des Hochfrequenzsignals durch Lokalspulen 50 ersetzt, die in dem Patiententunnel 16 nahe am Patient 100 angeordnet sind. Es ist aber grundsätzlich auch denkbar, dass die Lokalspule 50 zum Senden und Empfangen ausgelegt ist.

Eine Steuereinheit 20 versorgt die Magneteinheit 10 mit den Signalen für die Körperspule 14 und wertet die empfangenen Signale aus. Eine Magnetresonanztomographen-Steuerung 23 koordiniert dabei die Untereinheiten.

So weist die Steuereinheit 20 eine Gradientenansteuerung 21 auf, die dazu ausgelegt ist, die Gradientenspulen 12 über Zuleitungen mit variablen Strömen zu versorgen, welche zeitlich koordiniert die erwünschten Gradientenfelder in dem Untersuchungsvolumen bereitstellen.

Die Steuereinheit 20 weist eine Hochfrequenzeinheit 22 auf, die ausgelegt ist, einen Hochfrequenz-Puls mit einem vorgegebenen zeitlichen Verlauf, Amplitude und spektraler Leistungsverteilung zur Anregung einer Magnetresonanz der Kernspins in dem Patienten 100 zu erzeugen. Dabei können Pulsleistungen im Bereich von Kilowatt erreicht werden. Die einzelnen Einheiten sind über einen Signalbus 25 untereinander verbunden.

Das von Hochfrequenzeinheit 22 erzeugte Hochfrequenzsignal wird über eine Signalverbindung der Körperspule 14 zugeführt und in den Körper des Patienten 100 ausgesendet, um dort die Kernspins anzuregen. Denkbar sind auch gesonderte Sendespulen, die am Körper des Patienten angeordnet werden können.

Die Lokalspule 50 empfängt dann ein Magnetresonanzsignal aus dem Körper des Patienten 100, denn aufgrund des geringen Abstandes ist das Signal-zu-Rauschverhältnis (SNR) der Lokalspule 50 besser als bei einem Empfang durch die Körperspule 14. Das von der Lokalspule 50 empfangene MR-Signal wird in der Lokalspule 50 aufbereitet und an die Hochfrequenzeinheit 22 des Magnetresonanztomographen 1 zur Auswertung und Bilderfassung weitergeleitet.

Üblicherweise werden Magnetresonanzsignale von der Lokalspule über Kabel, vorzugsweise Koaxialkabel, zu dem Magnetresonanztomographen 1 übertragen. Die metallischen Leiter wirken jedoch auch als Antenne während der Anregungspulse. Um hohe induzierte Spannungen zu vermeiden sind deshalb Mantelwellensperren entlang der Kabel vorzusehen, damit der Patient nicht gefährdet wird. Um diese sperrigen Verbindungsleitungen zu vermeiden, werden zunehmend drahtlose Signalübertragungsverfahren betrachtet. Da die Lokalspule 50 aber auch aktive Elemente wie Vorverstärker, Wandler und Sender für die Übertragung aufweist, ist eine Energieversorgung ohne Drahtverbindung erforderlich.

Um die drahtlose Energieversorgung bereitstellen zu können, weist der Magnetresonanztomograph 1 eine Energiesendeeinrichtung 40 auf, mit der ein Wechselstrom erzeugt werden kann, der beispielsweise beim Fließen durch eine Sendespule 41 ein magnetisches Wechselfeld erzeugt. Vorzugsweise ist die Sendespule 41 dazu in der Nähe des Patiententunnels 16 angeordnet. Denkbar ist es auch, die Sendespule 41 in der Patientenliege 30 vorzusehen.

Ein möglichst großer Wechselstrom in der Sendespule 41 und damit starkes Wechselfeld sowie ein hoher Energietransfer in eine Induktionsspule 58 einer Lokalspule 50 lässt sich erzielen, wenn dabei die Sendespule 41 ein Teil eines Resonanzkreises ist, dessen Resonanzfrequenz die Frequenz des Wechselstromes der Energiesendeeinrichtung ist. Gemeinsam mit einem Resonanzkreis gleicher Frequenz in der Lokalspule 50 lässt sich ein besonders effektiver Energietransfer bereitstellen. Bei geeigneter Anpassung an den Erzeuger des Wechselstromes lässt sich auf vorteilhafte Weise auch eine unerwünschte Abstrahlung der Energie als elektromagnetische freie Welle reduzieren.

Sind jedoch zwei oder mehr Lokalspulen 50 mit Resonanzkreisen gleicher Frequenz in dem Patiententunnel 16, so treten diese über das magnetische Feld in Wechselwirkung. Gemäß der Theorie gekoppelter Schwingkreise tritt dann eine Aufspaltung der Resonanzfrequenzen auf, sodass keiner der Resonanzkreise mehr auf der ursprünglichen Resonanzfrequenz ist und der Energietransfer erheblich an Leistung verliert.

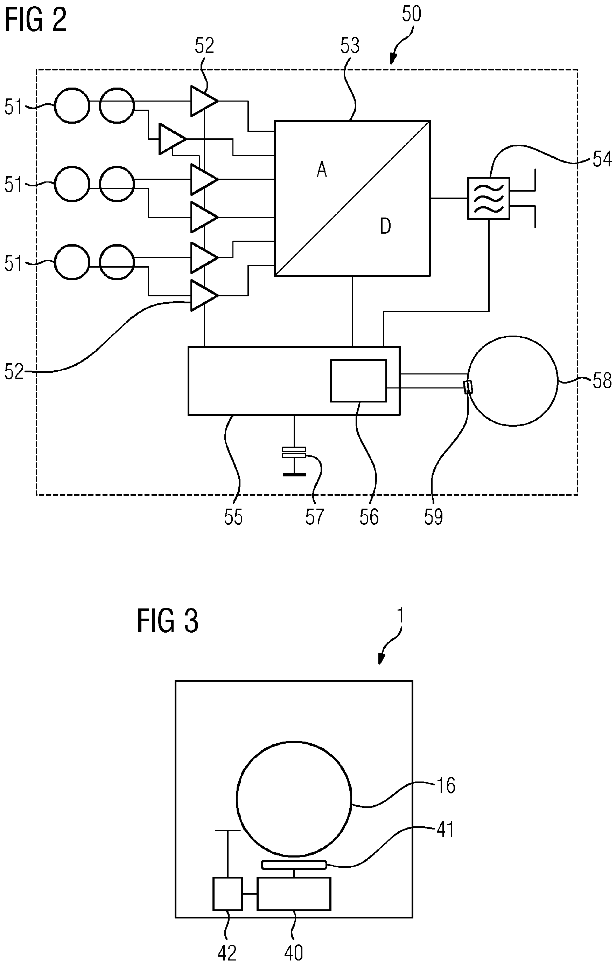

Die in Fig. 2 dargestellte Lokalspule 50 mit einer Energieversorgung 55 und einer Taktsteuerung 56 löst dieses Problem, indem die Taktsteuerung in Zusammenwirken mit einer Taktquelle 42 in dem Magnetresonanztomographen 1 dafür sorgt, dass die Resonanzkreise der Lokalspulen 50 nicht in Wechselwirkung treten. Die in der Fig. 2 gezeigte Lokalspule 50 kann im Rahmen der Erfindung verwendet werden, ist als solche jedoch nicht Teil der Erfindung.

Die Lokalspule 50 weist eine oder mehrere Antennenspulen 51 auf, in denen ein Magnetresonanzsignal aus dem Körper des Patienten 100 aufgenommen wird. Das Magnetresonanzsignal wird durch Vorverstärker 52, auch als Low Noise Amplifier LNA bezeichnet, verstärkt. In einer möglichen Ausführungsform wird das Magnetresonanzsignal direkt durch einen A/D-Wandler 53 digitalisiert. Es ist aber auch denkbar, dass das Magnetresonanzsignal zunächst in der Frequenz umgesetzt wird. Das Magnetresonanzsignal kann nach der Umsetzung auf eine oder mehrere Zwischenfrequenzen auch ohne AD-Wandlung als analoges Signal weiter übertragen werden. Zur drahtlosen Übertragung ist ein Sender 54 in der Lokalspule 50 vorgesehen, der das oder die analogen oder digitalen Signale auf eine Trägerwelle moduliert und über eine Antenne aussendet. Die Trägerwelle ist wegen der benötigten Bandbreite aller Magnetresonanzsignale vorzugsweise in einem Frequenzbereich von Gigahertz.

Die aktiven Komponenten wie Vorverstärker 52, AD-Wandler 53, Sender 54 oder mögliche, aber nicht dargestellte Einheiten wie Oszillatoren und Mischer benötigen zum Betrieb Energie in Form von elektrischem Strom. Die Versorgung erfolgt durch eine Energieversorgungseinrichtung 55 der Lokalspule 50. Die Energieversorgungseinrichtung 55 empfängt die Energie drahtlos über eine Energieempfangseinrichtung, beispielsweise über eine Induktionsspule 58 durch Wechselwirkung mit einem von einer Energiesendeeinrichtung 40 des Magnetresonanztomographen 1 erzeugten magnetischem Wechselfeld. Um die Energieübertragung möglichst effizient zu gestalten, ist die Induktionsspule 58 während der Energieübertragung resonant auf die Frequenz des magnetischen Wechselfeldes abgestimmt. Denkbar sind bei höheren Frequenzen aber auch beispielsweise Dipolantennen oder eine leitende Fläche bei einer kapazitiven Übertragung mittels elektrischer Wechselfelder.

Der durch das magnetische Wechselfeld induzierte Wechselstrom in der Induktionsspule wird anschließend in der Energieversorgungseinrichtung 55 gleichgerichtet und vorzugsweise in einem Energiespeicher 57 zwischengespeichert, um Unterbrechungen in der drahtlosen Energieübertragung zu überbrücken. Der Energiespeicher 57 kann dabei je nach zu überbrückender Zeit Kondensatoren, als Supercap bezeichnete elektrolytische Kondensatoren mit Kapazitäten bis zu Farad oder auch aufladbare Batterien wie beispielsweise Lithium-Ionen-, Lithium-Polymer- oder Lithium-Eisenphosphat Batterien aufweisen. Dabei ist es auch denkbar, dass in einer Kombination aus Supercap und Batterie der Supercap kurzfristig eine große Energie zwischenspeichert und dann in die Batterie abgibt, um einen zulässigen Ladestrom nicht zu überschreiten.

Die Energieversorgungseinrichtung 55 gibt dann den Strom kontinuierlich an die Verbraucher ab. Dabei ist denkbar, dass die Spannung durch eine störungsarme Längsregelung stabilisiert wird, oder ein Step-Up-Konverter mit besonderen Störunterdrückungsmaßnahmen die Spannung auch bei absinkendem Eingangswert konstant hält. Maßnahmen zur Störunterdrückung kann beispielsweise eine spezielle Frequenzwahl des Konverters oder ein Zusammenwirken mit der nachfolgend erläuterten Taktsteuerung umfassen.

Die Energieversorgungseinrichtung 55 weist weiterhin eine Taktsteuerung 56 auf. Die Taktsteuerung 56 ist ausgelegt, sich mit einer externen Taktquelle 42 zu synchronisieren. Dazu ist es beispielsweise denkbar, dass das magnetische Wechselfeld der Energiesendeeinrichtung 40 mit einer Synchroninformation moduliert ist. Ein Demodulator der Energieversorgungseinrichtung 55 kann das Synchronsignal demodulieren und der Taktsteuerung 56 zuführen. Denkbar ist aber auch die Übertragung separat auf einer anderen Frequenz. Beispielsweise kann eine schmalbandige drahtlose Signalverbindung zur Steuerung der Lokalspule 50 vorgesehen sein, mit der das Synchronsignal übertragen wird. Deren Frequenz kann auch im Bereich von wenigen Kilohertz oder Megahertz liegen. Möglich ist auch, dass die Taktsteuerungen 56 unterschiedlicher Lokalspulen 50 durch die Anregungspulse der Sequenzen als Synchroninformation synchronisiert werden.

Die Taktsteuerung 56 der Energieversorgungseinrichtung 55 der Lokalspule steht in Signalverbindung mit einer Verstimmeinrichtung 59 der Induktionsspule 58. Die Verstimmeinrichtung 59 ist dabei ausgelegt, die Induktionsspule 58 zu verstimmen, sodass diese weniger oder fast gar nicht mehr an das magnetische Wechselfeld der Energiesendeeinrichtung 40 ankoppelt und der drahtlose Energietransfer verringert oder gestoppt wird. Die Taktsteuerung 56 ist auf diese Weise in der Lage, die Energieaufnahme über die Energieversorgungseinrichtung zu steuern. Die Verstimmeinrichtung 59 kann beispielsweise eine Serienkapazität oder Induktivität aufweisen, die durch einen Schalter wie eine PIN-Diode von der Taktsteuerung kurzgeschlossen oder freigeschaltet werden kann. Denkbar sind auch andere Schaltungen wie parallele Reaktanzen mit entsprechenden Schaltern.

Durch die Taktsteuerung 56 in Verbindung mit der Synchronisierung erfolgt eine Verstimmung der Induktionsspule 58 immer derart, dass nicht alle Lokalspulen 50 gleichzeitig Energie empfangen. Vorzugsweise erfolgt eine Energieaufnahme zu jedem Zeitpunkt nur durch eine einzelne Lokalspule 50. Dadurch kann sichergestellt werden, dass die Induktionsspulen 58 unterschiedlicher Lokalspulen 50 nicht miteinander über das Magnetfeld wechselwirken und bei der Energieaufnahme mit unveränderter, optimaler Resonanzfrequenz möglichst effektiv Energie übertragen wird.

Denkbar ist die Synchronisierung der Taktsteuerung 56 beispielsweise derart, dass durch die Synchroninformation, beispielsweise einem Puls oder einem Pegelwechsel, Zähler in den Taktsteuerungen 56 aller Lokalspulen 50 synchronisiert werden. Es ist dann beispielsweise denkbar, dass die letzte Stelle einer Nummer bzw. einer Seriennummer einer Lokalspule 50 mit dem Zähler verglichen wird und eine Energieübertragung nur jeweils mit der Lokalspule 50 ausgeführt, deren letzte Stelle gerade mit dem Zählerstand übereinstimmt. Denkbar sind auch andere Algorithmen wie Modulo oder Bitoperationen. Die Nummer der jeweiligen Lokalspule 50 kann auch eingestellt oder ober einen Einstellbefehlt von dem Magnetresonanztomographen 1 vergeben werden, sodass Zugriffe zur gleichen Zeit ausgeschlossen und ungenutzte Zeitschlitze vermieden werden. Denkbar ist auch, dass die Synchroninformation unmittelbar die Nummer einer Lokalspule 50 enthält, die im nächsten Zeitschlitz laden darf, oder dass direkt ein Ladekommando mit der Synchroninformation an eine bestimmte Lokalspule 50 gesendet wird.

Möglich ist auch eine Lösung, bei der die Energieversorgungseinrichtung 55 über eine Kommunikationseinrichtung einen Ladezustand des Energiespeichers 57 an die Taktquelle 42 des Magnetresonanztomographen 1 weitergibt. Die Kommunikationseinrichtung kann beispielsweise eine schmalbandige drahtlose Kommunikationsschnittstelle sein, über die der Magnetresonanztomograph Statusinformationen oder Einstellkommandos mit der Lokalspule 50 austauscht. Denkbar ist aber auch, den Ladezustand beispielsweise mit digitalen Magnetresonanzsignalen zu multiplexen. In einer vorteilhaften Ausführungsform passt dann die Taktsteuerung 56 die Synchroninformation an den Ladezustand an, sodass beispielsweise die Lokalspule 50 mit dem niedrigsten Ladezustand bevorzugt geladen wird, indem sie mehr Zeitschlitze zugeordnet bekommt. Dies kann realisiert werden, indem die Nummer der Lokalspule 50 als Synchroninformation häufiger wiederholt wird oder direkte Ladekommandos von der Taktquelle 42 an die Lokalspule 50 gesendet werden.

In Fig. 3 ist noch die korrespondierende Energiesendeeinrichtung 40 des Magnetresonanztomographen 1 dargestellt. Die in der Fig. 3 gezeigte Energiesendeeinrichtung kann im Rahmen der Erfindung verwendet werden, ist jedoch als solche nicht Teil der Erfindung.

Der Magnetresonanztomograph 1 weist eine Energiesendeeinrichtung 40 auf, die ausgelegt ist, die Energie zur drahtlosen Energieversorgung bereitzustellen. Beispielsweise kann die Energiesendeeinrichtung 40 einen Oszillator und einen Leistungsverstärker aufweisen, mit dem ein hochfrequenter Wechselstrom erzeugt wird. Der hochfrequente Wechselstrom wird in eine Antenne, beispielsweise eine Sendespule 41 eingespeist, die in der Nähe des Patiententunnels 16 angeordnet ist. Durch eine gezielte Fehlanpassung zwischen Sendespule 41 und dem System aus Energiesendeeinrichtung und Zuleitung zur Sendespule 41 kann dabei erreicht werden, dass eine Energieübertragung nur durch Induktion im Nahfeld stattfindet, ohne größere Anteile der Sendeenergie als freie Radiowelle in den Raum abzustrahlen. Dadurch kann die SAR-Belastung reduziert und die regulatorische Zulassung vereinfacht werden.

Die Frequenz des von der Energiesendeeinrichtung 40 erzeugten Wechselstromes ist vorzugsweise so gewählt, dass Oberwellen nicht mit den Frequenzen des Magnetresonanzsignals oder einer in der nachfolgenden Auswertung verwendeten Zwischenfrequenzen zusammenfallen.

Die Taktquelle 42 kann mit der Energiesendeeinrichtung 40 in Signalverbindung stehen. Dabei ist die Energiesendeeinrichtung 40 ausgelegt, den hochfrequenten Wechselstrom mit einer Synchroninformation von der Taktsteuerung zu modulieren, sodass diese von der Energieversorgungseinrichtung 55 der Lokalspule 50 empfangen und an die Taktsteuerung 56 weitergegeben wird.

Es ist auch denkbar, dass die Synchroninformation von der Taktquelle 42 selbst über eine Antenne ausgesendet wird oder über einen Steuerkanal des Magnetresonanztomographen 1 zu der Lokalspule 50 drahtlos übertragen wird.

Die Taktquelle 40 kann ein einfacher Taktgeber sein oder auch eine komplexe Steuerung mit eigenem Prozessor, die eine von den Lokalspulen 50 übermittelte Information zu einem Ladezustand des Energiespeichers 57 auswertet und die Synchroninformation beispielsweise so anpasst, dass eine Lokalspule 50 mit niedrigem Ladezustand bevorzugt mit Energie versorgt wird.

Dabei ist es denkbar, dass die Taktquelle 42 als eigene Hardware ausgeführt ist. Ebenso ist es aber auch möglich, dass die Taktquelle 42 als Programm auf einer Steuerung des Magnetresonanztomographen 1 ausgeführt wird.

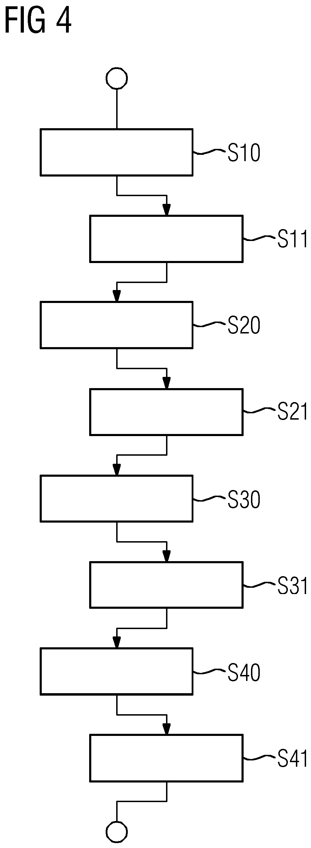

In Fig. 4 ist ein beispielhafter Ablaufplan eines Verfahrens dargestellt, dessen Verfahrensschritte zur Ausführung des in den Ansprüchen 3-7 definierten erfindungsgemäßen Verfahrens durchgeführt werden können. Das in der Fig. 4 gezeigte Verfahren ist jedoch als solches nicht Teil der Erfindung.

In einem Schritt S10 sendet die Taktquelle 42 eine Synchroninformation. Denkbar ist beispielsweise, dass die Synchroninformation über einen drahtlosen Steuerkanal des Magnetresonanztomographen gesendet wir, die Taktquelle 42 selbst einen Sender für ein drahtloses Senden aufweist oder auch das Signal der Energiesendeeinrichtung in einem Teilschritt S11 von der Taktquelle mit der Synchroninformation moduliert wird.

In einem Schritt S20 empfängt die Taktsteuerung 56 die Synchroninformation. Es ist dabei beispielsweise denkbar, dass die Energieversorgungseinrichtung 55 in einem Teilschritt S21 das von der Energiesendeeinrichtung 42 empfangene modulierte Signal mit dem Demodulator demoduliert und die empfangene Synchroninformation an die Taktsteuerung 56 weiterleitet.

In einem Schritt S30 nimmt die Energieversorgungseinrichtung 55 Energie über die Energieempfangseinrichtung in Abhängigkeit von der Synchroninformation auf. Die Energieempfangseinrichtung kann beispielsweise eine Induktionsspule 58 mit einer Verstimmeinrichtung 59 sein. Die Taktsteuerung 56 steuert dann in einem Teilschritt S31 direkt oder über die Energieversorgungseinrichtung 55 die Verstimmeinrichtung 59 derart an, dass Induktionsspule 58 resonant zu einem der Energiesendeeinrichtung erzeugten magnetischen Wechselfeld ist.

Es ist auch denkbar, dass in einem Schritt S40 die Energieversorgungseinrichtung 55 eine Information über einen Ladezustand des Energiespeichers 57 über die Kommunikationseinrichtung sendet, beispielsweise in einem Datenstrom gemultiplext mit den Magnetresonanzsignalen oder in einem gesonderten drahtlosen Rückkanal.

Vorzugsweise empfängt dann die Taktquelle 42 in einem Schritt S41 die Information über den Ladezustand und ändert die in Schritt S10 ausgesendete Synchroninformation in Abhängigkeit von der Information über den Ladezustand.

Obwohl die Erfindung im Detail durch die obigen Beispiele näher illustriert und beschrieben wurde, so ist die Erfindung nicht durch die offenbarten Beispiele eingeschränkt und andere Variationen können vom Fachmann hieraus abgeleitet werden, ohne den Schutzumfang der Erfindung zu verlassen, der durch die Ansprüche definiert wird.

## Patentansprüche

1. System aus einer Mehrzahl an Lokalspulen (50) für einen Magnetresonanztomographen (1), einer Energiesendeeinrichtung (40) und einer Taktquelle (42), wobei jede der Lokalspulen jeweils eine Energieversorgungseinrichtung (55) mit einem Energiespeicher (57), einer Taktsteuerung (56) und einer Energieempfangseinrichtung zum drahtlosen Empfang von Energie von der Energiesendeeinrichtung (40) aufweist, wobei die Taktsteuerung (56) ausgelegt ist, sich mit der Taktquelle (42) zu synchronisieren und die Taktsteuerung (56) in Signalverbindung mit der Energieversorgungseinrichtung (55) steht und ausgelegt ist, eine Energieaufnahme über die Energieempfangseinrichtung in Abhängigkeit von der Synchronisierung zu steuern, **dadurch gekennzeichnet, dass** die Taktquelle (42) ausgelegt ist, eine Synchroninformation derart an die Taktsteuerungen (56) der Lokalspulen (50) zu senden, dass nur eine echte Teilmenge der Lokalspulen (50) gleichzeitig Energie über die Energieempfangseinrichtung aufnimmt.

2. System nach Anspruch 1, wobei die Taktquelle (42) in Signalverbindung mit der Energiesendeeinrichtung (40) steht und ausgelegt ist, eine Energieaussendung der Energiesendeeinrichtung (40) zu modulieren.

3. Verfahren zum Betrieb eines Systems nach einem der Ansprüche 1 oder 2, wobei das Verfahren die Schritte aufweist:
Senden (S10) einer Synchroninformation durch die Taktquelle (42) ;
Empfangen (S20) der Synchroninformation durch die Taktsteuerungen (56) der Lokalspulen (50), Energieaufnahme (S30) durch die jeweilige Energieversorgungseinrichtung (55) über die jeweilige Energieempfangseinrichtung in Abhängigkeit von der Synchroninformation.

4. Verfahren nach Anspruch 3, wobei die Energieempfangseinrichtung von wenigstens einer der Lokalspulen (50) eine Induktionsspule (58) mit einer Verstimmeinrichtung (59) aufweist und die zugehörige Taktsteuerung (56) ausgelegt ist, die Verstimmeinrichtung (59) zu steuern, wobei der Schritt der Energieaufnahme (S30) den Schritt (S31) aufweist, den Zustand der Verstimmeinrichtung (59) zu ändern.

5. Verfahren nach Anspruch 3 mit einem System nach Anspruch 2, wobei die Taktsteuerung (56) von wenigstens einer der Lokalspulen (50) einen Synchronisierungseingang zum Synchronisieren mit der Taktquelle (42) aufweist, wobei der Synchronisierungseingang in Signalverbindung mit der Energieempfangseinrichtung steht und einen Demodulator aufweist, um ein von der Energieempfangseinrichtung (55) empfangenes Synchronsignal zu demodulieren, wobei der Schritt des Sendens (S10) den Schritt (S11) aufweist, die Energieaussendung der Energiesendeeinrichtung (40) mit der Synchroninformation zu modulieren und der Schritt (S20) des Empfangens den Schritt (S21) aufweist, das von der Energiesendeeinrichtung (42) empfangene Signal mit dem Demodulator zu demodulieren und die empfangene Synchroninformation an die Taktsteuerung (56) weiterzuleiten.

6. Verfahren nach Anspruch 3, wobei die Taktsteuerung (56) von wenigstens einer der Lokalspulen (50) in Signalverbindung mit einer drahtlosen Kommunikationseinrichtung steht und die Taktsteuerung (56) oder die Energieversorgungseinrichtung (55) der wenigstens einen Lokalspule (50) ausgelegt sind, eine Information über einen Ladezustand des Energiespeichers (57) über die Kommunikationseinrichtung zu senden, wobei das Verfahren den Schritt (S40) aufweist, eine Information über einen Ladezustand des Energiespeichers (57) über die Kommunikationseinrichtung zu senden.

7. Verfahren nach Anspruch 6, wobei die Taktquelle (42) die Information über den Ladezustand in einem Schritt (S41) empfängt und eine im Schritt (S10) des Sendens ausgesendete Synchroninformation in Abhängigkeit von der Information über den Ladezustand ändert.

## Claims

1. System comprising a plurality of local coils (50) for a magnetic resonance tomography system (1), an energy transmitting device (40) and a clock source (42),
wherein each of the local coils has in each case an energy supply device (55) with an energy store (57), a clock control system (56) and an energy receiving device for the wireless reception of energy from the energy transmitting device (40), wherein the clock control system (56) is configured to be synchronised with the clock source (42) and the clock control system (56) has a signal connection with the energy supply device (55) and is configured to control energy take-up via the energy receiving device in dependence on the synchronisation,
**characterised in that**,
the clock source (42) is configured to transmit synchronisation information to the clock control systems (56) of the local coils (50) such that only a genuine subset of the local coils (50) simultaneously takes up energy via the energy receiving device.

2. System according to claim 1, wherein the clock source (42) has a signal connection with the energy transmitting device (40) and is configured to modulate energy emission from the energy transmitting device (40).

3. Method for operating a system according to one of claims 1 or 2, wherein the method comprises the steps:
transmitting (S10) synchronisation information by the clock source (42);
reception (S20) of the synchronisation information by the clock control systems (56) of the local coils (50);
energy take-up (S30) by the respective energy supply device (55) via the respective energy receiving device in dependence on the synchronisation information.

4. Method according to claim 3, wherein the energy receiving device comprises an induction coil (58) from at least one of the local coils (50) with a detuning device (59) and the associated clock control system (56) is configured to control the detuning device (59), wherein the step (S30) of energy take-up comprises the step (S31) of changing the status of the detuning device (59).

5. Method according to claim 3 with a system according to claim 2, wherein the clock control system (56) comprises a synchronisation input from at least one of the local coils (50) for synchronisation with the clock source (42), wherein the synchronisation input has a signal connection with the energy receiving device and a demodulator in order to demodulate a synchronising signal received from the energy receiving device (55),
wherein the step (S10) of transmitting comprises the step (S11) of modulating the energy emission of the energy transmitting device (40) with the synchronisation information and the step (S20) of receiving comprises the step (S21) of demodulating the signal received from the energy transmitting device (42) with the demodulator and forwarding the received synchronisation information to the clock control system (56).

6. Method according to claim 3, wherein the clock control system (56) from at least one of the local coils (50) has a signal connection with a wireless communication device and the clock control system (56) or the energy supply device (55) of the at least one local coil (50) are configured to transmit information on a state of charge of the energy store (57) via the communication device,
wherein the method has the step (S40) of transmitting information on a state of charge of the energy store (57) via the communication device.

7. Method according to claim 6, wherein the clock source (42) receives the information on the state of charge in one step (S41) and changes the synchronisation information emitted in step (S10) of transmitting in dependence on the information on the state of charge.

## Revendications

1. Système composé d'une pluralité de bobines (50) locales pour un tomodensitomètre (1) à résonnance magnétique, d'un dispositif (40) d'envoi d'énergie et d'une source (42) d'horloge, dans lequel chacune des bobines locales a respectivement un dispositif (55) d'alimentation en énergie, ayant un accumulateur (57) d'énergie, une commande (56) d'horloge et un dispositif de réception d'énergie pour la réception sans fil d'énergie du dispositif (40) d'envoi d'énergie, dans lequel la commande (56) d'horloge est conçue pour se synchroniser avec la source (42) d'horloge et la commande (56) d'horloge est en liaison de signal avec le dispositif (55) d'alimentation en énergie et conçue pour commander une absorption d'énergie par le dispositif de réception d'énergie en fonction de la synchronisation, **caractérisé en ce que**
la source (42) d'horloge est conçue pour envoyer une information de synchronisation aux commandes (56) d'horloge des bobines (50) locales,
de manière à ce que seul un ensemble partiel vrai des bobines (50) locales absorbe en même temps de l'énergie par le dispositif de réception d'énergie.

2. Système suivant la revendication 1, dans lequel la source (42) d'horloge est en liaison de signal avec le dispositif (40) d'envoi d'énergie et est conçue pour moduler une émission d'énergie du dispositif (40) d'envoi d'énergie.

3. Procédé pour faire fonctionner un système suivant l'une des revendications 1 ou 2, dans lequel le procédé a les stades :
Envoi (S10) d'une information de synchronisation par la source (42) d'horloge ;
Réception (S20) de l'information de synchronisation par les commandes (56) d'horloge des bobines (50) locales ;
Absorption (S30) d'énergie par le dispositif (55) respectif d'alimentation en énergie par l'intermédiaire du dispositif respectif de réception d'énergie en fonction de l'information de synchronisation.

4. Procédé suivant la revendication 3, dans lequel le dispositif d'absorption d'énergie d'au moins l'une des bobines (50) locales a une bobine (58) d'induction ayant un dispositif (59) d'accord et la commande (56) d'horloge associée est conçue pour commander le dispositif (59) d'accord, le stade d'absorption (S30) d'énergie ayant le stade (S31) de modification de l'état du dispositif (59) d'accord.

5. Procédé suivant la revendication 3, par un système suivant la revendication 2,
dans lequel la commande (56) d'horloge d'au moins l'une des bobines (50) locales a une entrée de synchronisation pour la synchronisation avec la source (42) d'horloge, dans lequel l'entrée de synchronisation est en liaison de signal avec le dispositif de réception d'énergie et a un démodulateur pour démoduler un signal de synchronisation reçu du dispositif (55) de réception d'énergie,
dans lequel le stade de l'envoi (S10) a le stade (S11) de modulation de l'émission d'énergie du dispositif (40) d'envoi d'énergie par l'information de synchronisation et le stade (S20) de la réception a le stade (S21) de démodulation par le démodulateur du signal reçu du dispositif d'envoi d'énergie et d'acheminement à la commande (56) d'horloge de l'information de synchronisation reçue.

6. Procédé suivant la revendication 3, dans lequel le dispositif (56) d'horloge d'au moins l'une des bobines locales est en liaison de signal avec un dispositif de communication sans fil et la commande (56) d'horloge ou le dispositif (55) d'alimentation en énergie de la au moins une bobine (50) locale sont conçus pour envoyer par le dispositif de communication une information sur l'état de charge de l'accumulateur (57) d'énergie,
dans lequel le procédé a le stade (S40) d'envoi par le dispositif de communication d'une information sur l'état de charge de l'accumulateur (57) d'énergie.

7. Procédé suivant la revendication 6, dans lequel la source (42) d'horloge reçoit dans un stade (S41) l'information sur l'état de charge et modifie, en fonction de l'information sur l'état de charge, une information de synchronisation émise dans le stade (S10) de l'envoi.
